(19) Europäisches Patentamt
European Patent Office
Office européen des brevets

(11) Veröffentlichungsnummer: **0 088 446**
**A2**

(12) **EUROPÄISCHE PATENTANMELDUNG**

(21) Anmeldenummer: **83102314.8**

(22) Anmeldetag: **09.03.83**

(51) Int. Cl.³: **C 07 D 407/14**, C 07 H 7/00, C 12 P 17/16, A 61 K 31/335 // A23K1/17 ,(C12P17/16, C12R1/465)

(30) Priorität: **10.03.82 US 356654**

(43) Veröffentlichungstag der Anmeldung: **14.09.83 Patentblatt 83/37**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Liu, Chao-Min, 36 Rockledge Place, Cedar Grove N.J. 07009 (US)**
Erfinder: **Tresner, Homer D., Munson Road Route 1, La Farge Wis. 54639 (US)**
Erfinder: **Westley, John, 91 South Mountain Avenue, Cedar Grove N.J. 07009 (US)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

(54) **Neue Streptomyces-Antibiotika, Verfahren zu deren Herstellung sowie deren Verwendung.**

(57) Es werden die Antibiotika X-14873A, G und H der Formel

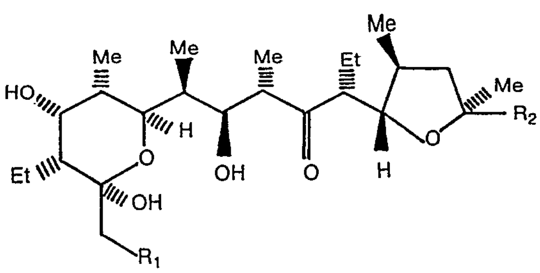

(I)

wobei für X-14873A $R_1$ $CO_2H$ und $R_2$

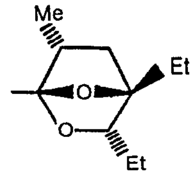

ist,
für X-14873G $R_1$ Wasserstoff und $R_2$

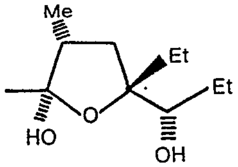

ist,
und für X-14873H $R_1$ Wasserstoff und $R_2$

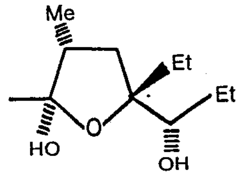

ist,
und wobei Me für Methyl und Et für Äthyl steht. Diese Verbindungen zeigen antibiotische Aktivität. Es wird ebenfalls ein Verfahren zur Herstellung dieser Verbindungen beschrieben.

EP 0 088 446 A2

0088446

EP-57 029

RAN 4410/162

# Neue Streptomyces-Antibiotika, Verfahren zu deren Herstellung sowie deren Verwendung

Die vorliegende Erfindung betrifft die Antibiotika X-14873A, G und H der Formel

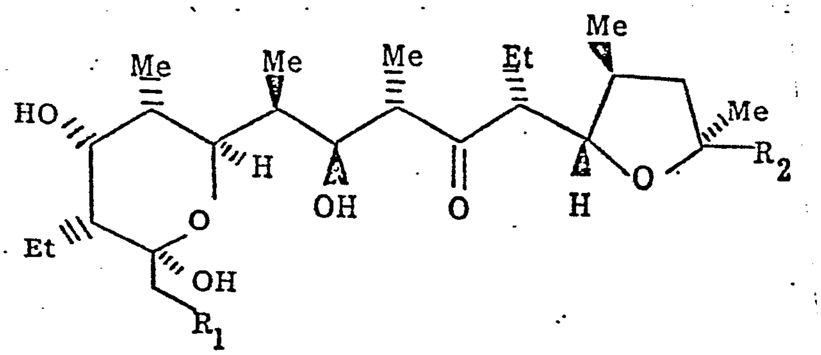

I

worin für X-14873A $R_1$ $CO_2H$ und $R_2$ die Gruppe

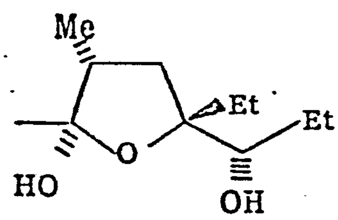

;

für X-14873G, $R_1$ Wasserstoff und $R_2$ die Gruppe

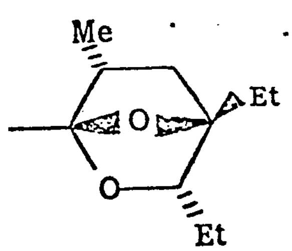

;

Klt/ 21.2.83

und für X-14873H, $R_1$ Wasserstoff und $R_2$ die Gruppe

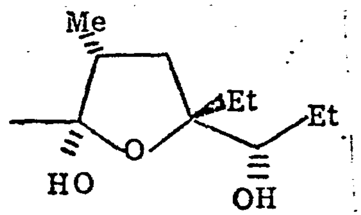

bedeutet.

Von der vorliegenden Erfindung werden auch die pharmazeutisch annehmbaren Salze dieser Antibiotika umfasst.

In den vorstehenden Strukturformeln bedeutet der Ausdruck Me Methyl und Et Aethyl.

Die vorliegende Erfindung umfasst weiterhin ein Fermentationsverfahren für die Herstellung dieser Antibiotika sowie Isoliertechniken, um die Antibiotika aus der Fermentationsbrühe zu gewinnen. Weiterhin wird die Isolierung von drei neuen Actinomycinen nämlich X-14873B, C und D beschrieben.

Der die erfindungsgemässen Antibiotika erzeugende Organismus ist eine neue Spezies mit der Bezeichnung Streptomyces sp. X-14873. Eine Kultur des lebenden Organismus mit der Laborbezeichnung X-14873 wurde bei American Type Culture Collection, Rockville, Maryland hinterlegt und deren permanenten Sammlung von Mikroorganismen als ATCC 31679 hinzugefügt. Der neue Mikroorganismus wurde aus einer Probenprobe in der Nähe von Sagebrush in Crandall Creek, Wyoming, isoliert.

<u>Wachstumskennzeichen</u>:

Der Mikroorganismus wurde auf Standard Difco ISP-Medien gezüchtet, wie sie in Shirling and Gottlieb: "Methods for Characterization of Streptomyces Species", Intern. J. System. Bacteriol., 16, Seiten 313-340, 1966, beschrieben sind.

Für weitere Tests verwendete Medien werden in den nachstehenden Publikationen beschrieben:

| Test | Publikationen |
| --- | --- |
| A. Kochsalzverträglichkeit<br>B. Caseinhydrolyse<br>C. Nitratreduktion | Gordon and Smith "Rapidly Growing Acid Fast Bacteria", J. Bacteriol., 66:41-48, 1953 |
| D. Gelatine (modifiziert mit Nährgelatine [BBL] plus 2,00% Agar anstelle von Fleischinfusionsagar)<br>E. Stärke (Actinomycesmedium [Difco] plus 0,25% lösliche Stärke und 2,0% Agar) | Skerman, "A Guide to the Identification of the Genera of Bacteria", Williams and Williams Co., Baltimore, 1967. |
| F. Abbau von Adenin, Xanthin, Tyrosin und Hypoxanthin | Gordon, "The taxonomy of soil bacteria", S. 293-321, Gray and Parkinson, Ecology of Soil Bacteria, Liverpool Press, Liverpool, England. |

Die Versuche werden bei 28°C und 37°C für fast sämtliche Medien durchgeführt. Farbbestimmungen wurden nach 2- und 4-wöchiger Inkubation durchgeführt. Die Pigmentierung wurde unter Verwendung des Farbschemas im Color Harmony Manual, 4. Ausgabe, 1958, beschrieben.

Die Kohlenstoffverwertung wurde nach der Methode von Shirling and Gottlieb (oben) mit Hilfe von ISP-9 (Difco) Medium bestimmt.

Zwecks Erhaltung einer gewaschenen Suspension für Beimpfung wurde eine 48-Stunden alte ISP-1 Brühekultur von X-14873 zentrifugiert und homogenisiert.

Die Fähigkeit des Organismus, bei 10°, 28°, 37°, 45° und 50°C zu wachsen, wurde durch Beimpfung von ISP-1 (Difco) Medium untersucht. Zellwandanalyse des Isomeren von Diaminopimelinsäure wurde nach der Methode von Becker et al., Appl. Microbiol., 12, 421-423, 1964, durchgeführt.

## Ergebnisse

Mikroskopische Untersuchung:

Der Stamm X-14873 erzeugt ein Substratmycel, welches nicht in Sporen und Luftmycel fragmentiert, sondern rectus flexibilis Sporenketten mit 10-20 Sporen pro Kette bildet. Die Sporen sind glatt und bewegen sich in der Grössenordnung von 1,0 x 0,52 µm bis 1,25 bis 0,75 µm.

Zellwandanalyse:

Die Zellwand enthält das LL-Isomere von Diaminopimelinsäure, was, zusammen mit dem oben erwähnten mikroskopischen Untersuchungen, diesen Mikroorganismus in die Gattung Streptomyces einordnet.

Makroskopische Untersuchungen:

Tabelle 1 fasst das Wachstumsverhalten, den Sporulationsgrad, die Phase der Sporenmasse, die Farbe der Rückseite des Mycels und die Anwesenheit von durch X-14873 auf verschiedenen Agarmedien erzeugtem Pigment zusammen.

Tabelle 1

Kulturmerkmale des Stammes X-14873

| Agarmedium | Wachstums- und Sporulationsgrad | Farbe der Sporenmasse | Farbe der Rückseite des Mycels |
|---|---|---|---|
| Hefe-Malzextrakt (ISP 2) | mittleres bis reichliches Wachstum; mittlere Sporulation; braun lösliches Pigment | c (hellgrau) wenn sporuliert; 3ni (nelkenbraun) wo nicht sporuliert | 3ie (kamelfarben) und 2gc (gelbbraun) an den Ecken |
| Hafermehl (ISP 3) | mittleres Wachstums; spärliche Sporulation; gelb lösliches Pigment | b (austernweiss) wo sporuliert; 2gc (bambusfarben) wo nicht sporuliert | 2ec (biskuitfarben) |
| Anorganische Salze/Stärke (ISP 4) | reichliches Wachstum; gute Sporulation; hydrolysierte Stärke; gelb lösliches Pigment | 3cb (sandfarben) | 31c (bernsteinfarben) und 31e (zimtfarben) |
| Glycerin-Asparagin (ISP 5) | mittleres Wachstum; mittlere Sporulation; gelb braunes lösliches Pigment; hygroskopisch | b (austernweiss) | 31e (zimtfarben) |
| Czapek-Dox | mittleres Wachstum; mittlere Sporulation; gelb lösliches Pigment; leicht hygroskopisch | b (austernweiss) | 2ic (honiggelb) |

Physiologische Kennzeichen:

Der Mikroorganismus X-14873 hydrolysiert Casein, Stärke, Gelatin, Adenin, Xanthin, Hypoxanthin, Tyrosin und Harnstoff. Tabelle 2 vergleicht die Kohlenstoffverwertung des Stammes X-14873 mit denen von Streptomyces chrysomallus, Streptomyces parvus und Streptomyces globisporus, welche im Hinblick auf ihre Aehnlichkeiten in den morphologischen und physiologischen Eigenschaften ausgewählt wurden.

Tabelle 2

Vergleich der Kohlenstoffverwertung durch Stamm X-14873 und verwandte Stämme

| | Ergebnis[a] | | | |
|---|---|---|---|---|
| Kohlenstoffquelle | | S.chrysomallus | S. parvus | S.globisporus |
| | X-14873 | 3194A | 3195A | 3201A |
| Kontrollprobe (kein Kohlenstoff) | - | - | - | - |
| D.Glucose | ++ | ++ G | ++ G | ++ G |
| D-Xylose | ± | + | ++ G | + |
| L-Arabinose | + | + | +(+) | + |
| L-Rhamnose | ++ G | ++ G | ++ | ++ |
| D-Fructose | ++ | ++ | ++ | ++ |
| D-Galactose | ++ | ++ G | ++ G | ++ |
| Raffinose | ± | - | - | - |
| D-Mannit | ++ G | ++ G | ++ G | ++ |
| i-Inosit | ± | - | - | - |
| Salicin | + | ± | ± | ± |
| Sucrose | - | - | - | - |
| Cellulose | - | - | - | - |
| Maltose | - | ++ G | ++ intensiv G | ++ |
| Glycerin | ++ | ++ | ++ intensiv G | ++ |
| Stärke | ++ | ++ G | ++ | ++ |
| Ribose | ++ | ++ G | ++ G | ++ G |

[a]-: Negative Reaktion; ±: Zweifelhafte Reaktion; +: Kräftigeres Wachstum als auf Kontrollprobe, aber weniger als auf Glucose; +(+): Positive Reaktion, fast gleichwertig mit dem Wachstum auf Glucose; ++: Positive Reaktion gleichwertig mit dem Wachstum auf Glucose. G = gelb lösliches Pigment.

In Tabelle 3 sind einige metabolische und morphologische Eigenschaften zusammengestellt.

Tabelle 3

| Test | Ergebnis |
|---|---|
| Farbe der Sporenmasse | grau (gelblich) |
| ISP 6, Verdunkelung | - |
| Melanin, ISP 7 | - |
| ISP 1, Verdunkelung | - |
| Hydrolyse von Casein | + |
| Hydrolyse von Stärke | + |
| NaCl-Toleranz (%) | 5 |
| Temperatur, bei der Wachstum beobachtet wurde | 10-28° |
| Pigment der Rückseite | - |
| Lösliches Pigment | gelb |
| Streptomycin-Empfindlichkeit (10 mcg in 1/4" Scheibe) | + 13 mm |
| Lysozym-Empfindlichkeit | + |
| Nitratreduktion | + |
| Hygroskopische Eigenschaft | + |
| Antibiotika-Produktion | Antibiotikum X-14873A |
| | " X-14873G |
| | " X-14873H |
| | Actinomycine X-14873B |
| | " X-14873C |
| | " X-14873D |

Streptomyces Stamm X-14873 gleicht S. chrysomallus, S. parvus und S. globisporus. Alle ausser S. globisporus erzeugen Actinomycin. Die drei bekannten Kulturen wurden auch auf die Erzeugung von Hydroxylsocellin untersucht. In einem Bioautogram gegen Bacillus sp. (ATCC 27860) erzeugten sowohl S. chrysomallus wie S. parvus eine Aktivität bei ähnlichen $R_F$ in zwei unterschiedlichen Lösungsmittelsystemen.

Die bekannten Kulturen besitzen eine gelbgefärbte Sporenmasse, wogegen X-14873 hauptsächlich grau ist und nur eine Andeutung zu gelb hat. Stamm X-14873 erzeugt ein gelbes lösliches Pigment wie dies die andern Stämme tun. Da diese Kulturen alle Stärke, Gelatine, Casein und Harnstoff hydrolysieren und Adenin, Xanthin, Hypoxanthin und Tyrosin zersetzen, ist eine Unterscheidung zwischen diesen Kulturen schwierig.

Stamm X-14873 kann nicht einer bestimmten Spezies zugeordnet werden, sondern gleicht einer Grupee, welche Streptomyces chrysomallus - S. parvus Gruppe genannt werden könnte und zwar basierend auf der Aehnlichkeit in der Antibiotika-Produktion und in der Erzeugung von löslichen gelben Pigment sowie im Kohlenstoffverwertungsverhalten.

Der Stamm Streptomyces X-14873, wie er hierin beschrieben wird, umfasst alle Stämme von Streptomyces, welche die Verbindungen X-14873A, B, C, D, G und H erzeugen und welche nicht eindeutig von der Kultur X-14873 und deren Subkulturen einschliesslich Mutanten und Varianten davon unterschieden werden können.

Streptomyces sp. X-14873 erzeugt unter geeigneten Bedingungen eine Verbindung der Formel I. Eine Fermentationsbrühe enthaltend Streptomyces X-14873 wird durch Beimpfen eines geeigneten Mediums mit Sporen oder Mycel des Organismus, welcher die Verbindung der Formel I erzeugt, erhalten;

die Nährlösung wird anschliessend unter aeroben Bedingungen kultiviert. Es ist ebenfalls möglich, die Kultivierung auf einem festen Nährmedium durchzuführen, doch wird für die Erzeugung grösserer Mengen ein flüssiges Medium bevorzugt. Die Gärtemperatur bei der der Organismus wächst, kann über einen weiten Bereich, z.B. 20-35°C variiert werden, doch ist eine Temperatur von 26-30°C und ein im wesentlichen neutrales pH bevorzugt. Für die submerse, aerobe Fermentation des Organismus zur Erzeugung einer Verbindung der Formel I kann das Nährmedium als Kohlenstoffquelle ein im Handel erhältliches Glyceridöl oder ein Kohlehydrat, wie Glycerin, Glucose, Maltose, Lactose, Dextrin, Stärke, etc., in reinem oder rohem Zustand enthalten; als Stickstoffquelle kommen organische Materialien, wie z.B. Soyabohnenmehl, lösliche Rückstände aus der Brennerei, Erdnussöl, Baumwollsamenmehlextrakt, Pepton, Fischmehl, Hefeextrakt, Maisquellwasser etc., in Betracht. Erwünschtenfalls können auch anorganische Stickstoffquellen verwendet werden, wie z.B. Nitrate und Ammoniumsalze und Mineralsalze, wie Ammoniumsulfat, Magnesiumsulfat und dergleichen. Das Medium kann auch Natriumchlorid, Kaliumchlorid, Kaliumphosphat und dergleichen und Puffer wie Natriumcitrat, Calciumcarbonat oder -phosphat und Spurenelemente von Schwermetallsalzen. Wenn die Fermentation unter belüfteten submersen Bedingungen durchgeführt wird, verwendet man vorzugsweise ein Schaumbekämpfungsmittel, wie flüssiges Paraffin, fettes Oel oder Silikonprodukte. Mehr als eine Art von Kohlenstoffquelle, Stickstoffquelle oder Schaumbekämpfungsmittel kann verwendet werden.

Ebenfalls Gegenstand der vorliegenden Erfindung sind die organischen oder anorganischen, pharmazeutisch annehmbaren Salze einer Verbindung der Formel I. Diese Salze werden aus der freien Säure nach bekannten Methoden hergestellt, z.B. durch Waschen der freien Säure in Lösung mit einer geeigneten Base oder einem Salz. Beispiele für solche pharmazeutisch annehmbare basische Substanzen, die zur Salzbildung fähig sind, umfassen Alkalimetallbasen, wie

Natriumhydroxid, Kaliumhydroxid, Lithiumhydroxid und Erdalkalimetallbasen, wie Calciumhydroxid, Bariumhydroxid und dergleiche, und Ammoniumhydroxid. Alkalimetall- oder Erdalkalimetallsalze, welche verwendet werden können, sind beispielsweise Carbonate, Bicarbonate und Sulfate. Im Rahmen der vorliegenden Erfindung sind Salze, welche von Alkalimetallbasen gebildet wurden, bevorzugt.

Beispiele organischer Basen, welche pharmazeutisch annehmbare Salze mit den Verbindungen der Formel I bilden, sind niedere Alkylamine, primäre, sekundäre und tertiäre Hydroxy(nieder)alkylamine, Aethylamin, Isopropylamin, Diäthylamin, Methyl-n-butylamin, Aethanolamin und Diethanolamin.

Die folgenden Beispiele dienen zur Illustration der Erfindung ohne diese zu limitieren.

## Beispiel 1

### Schüttelkolbengärung

Die Streptomyces X-14873-Kultur wird auf einer Stärke-Casein-Agarschrägkultur folgender Zusammensetzung (g/l in destilliertem Wasser) gezüchtet und aufbewahrt:

| | |
|---|---|
| Lösliche Stärke | 10,0 |
| Casein | 1,0 |
| $K_2HPO_4$ | 0,5 |
| $MgSO_4$ (wasserfrei) | 0,5 |
| Agar | 20,0 |

Der pH-Wert wird vor dem Autoklavieren mit NaOH auf 7,4 eingestellt.

Die Schrägkultur wird mit X-14873-Kultur beimpft und bei 28° während 7-14 Tagen inkubiert. Ein Teil des Agars enthaltend Sporen und Mycel der gut wachsenden Kultur wird zur Beimpfung eines 500 ml-Erlenmeyer-Kolbens mit 100 ml Impfsubstrat der folgenden Zusammensetzung (g/l in destilliertem Wasser) verwendet:

| | |
|---|---|
| Tomatenmark | 5,0 |
| getrocknete Rückstände aus der Brennerei | 5,0 |
| OM Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird vor der Sterilisierung auf 7 eingestellt.

Die beimpfte Nährlösung wird bei 28° während 72 Stunden auf einer Schüttelmaschine (250 Upm) inkubiert.

Eine 30 ml-Portion der erhaltenen Kultur wird zur Beimpfung eines 6 l-Erlenmeyerkolbens enthaltend 1,25 l sterilisiertes Nährmedium folgender Zusammensetzung (g/l destilliertes Wasser) verwendet:

| | |
|---|---|
| Tomatenmark | 5,0 |
| getrocknete Rückstände aus der Brennerei | 5,0 |
| OM Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird vor der Sterilisation auf 7 eingestellt.

Die beimpfte Nährlösung wird bei 28° während 5 Tagen auf einer Schüttelmaschine (250 Upm) inkubiert.

## Beispiel 2

### Tankgärung

Die Streptomyces X-14873-Kultur wird auf einer Stärke-Casein-Agarschrägkultur folgender Zusammensetzung (g/l destilliertes Wasser) gezüchtet und aufbewahrt:

| | |
|---|---|
| Lösliche Stärke | 10,0 |
| Casein | 1,0 |
| $K_2HPO_4$ | 0,5 |
| $MgSO_4$ | 0,5 |
| Agar | 20,0 |

Der pH-Wert wird vor dem Autoklavieren mit Natriumhydroxid auf 7,4 gestellt.

Die Schrägkultur wird mit X-14873-Kultur beimpft und bei 28° während 7-14 Tagen inkubiert. Ein Teil des Agars enthaltend Sporen und Mycel aus der gut wachsenden Agarkultur wird zur Beimpfung eines 500 ml-Erlenmeyerkolbens enthaltend 100 ml sterilisiertes Impfsubstrat folgender Zusammensetzung (g/l in destilliertem Wasser) verwendet:

| | |
|---|---|
| Tomatenmark | 5,0 |
| getrocknete Rückstände aus der Brennerei | 5,0 |
| OM Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird mit Natriumhydroxid vor der Sterilisation auf 7,0 gestellt.

Die beimpfte Nährlösung wird während 72 Stunden bei 28° auf einer Schüttelmaschine (250 Upm) inkubiert.

20 ml (1%, V/V) dieser Kultur wird zur Beimpfung eines 6 l-Erlenmeyerkolbens enthaltend 2 l Medium der folgenden Zusammensetzung (g/l in destilliertem Wasser) verwendet:

| | |
|---|---|
| Tomatenmark | 5,0 |
| getrocknete Rückstände aus der Brennerei | 5,0 |
| OM Pepton | 5,0 |
| entbitterte Hefe | 5,0 |
| Maisstärke | 20,0 |
| $CaCO_3$ | 1,0 |
| $K_2HPO_4$ | 1,0 |

Der pH-Wert wird auf 7,0 eingestellt; anschliessend wird 45 Minuten bei 1-1 1/2 $kg/cm^2$ autoklaviert.

Eine 4 l-Portion dieser Kultur wird zur Beimpfung von 230 l Nährmedium folgender Zusammensetzung (g/l Leitungswasser) in einem 380 l Fermenter verwendet:

| | |
|---|---|
| Tomatenmark | 5,0 |
| getrocknete Rückstände aus der Brennerei | 5,0 |
| Pepton | 5,0 |
| entbitterte Hefe | 5,0 |
| Maisstärke | 20,0 |
| $CaCO_3$ | 1,0 |
| $K_2HPO_4$ | 1,0 |
| SAG 4130 Antischaummittel (Union Carbide) | 0,1 |

Der pH des Mediums wird vor der Sterilisation mit Natriumhydroxid auf 7,0 gestellt. Anschliessend wird 1 1/4 Stunden mit Dampf von 4,2 $kg/cm^2$ sterilisiert.

Das beimpfte Medium wird mit Druckluft mit einer Geschwindigkeit von 90 l/Minute belüftet und mit einem Rührwerk (280 Upm) gerührt. Die Gärung wird bei 28° während 5 Tagen durchgeführt.

## Beispiel 3

Isolierung des Natriumsalzes des Antibiotikums X-14873A aus der Schüttelkolbengärung gemäss Beispiel 1.

Stufe A. Die ganze Gärlösung von vier 6 l-Erlenmeyerkolben, von denen jeder 1,25 l enthält, wird nach 5 Tagen Gärung zweimal mit einem gleichen Volumen von Essigester extrahiert. Nach halbstündigem Rühren wird die Lösungsmittelschicht abgetrennt und unter vermindertem Druck zu einem Oel (3 g) konzentriert. Das Oel wird in Methylenchlorid gelöst und an einer 150 g Kieselgelkolonne chromatographiert. Die Kolonne wird mit einem Gradienten zwischen 4 l Methylenchlorid bis 4 l Hexan/Aceton (7/3) und

dann 3 l Methylenchlorid/Methylalkohol (8/2) eluiert. Es werden Fraktionen von 40 ml gesammelt und die Fraktionen Nrn. 181-480 werden gepoolt. Das Lösungsmittel wird unter vermindertem Druck entfernt und der erhaltene Rückstand (1,2 g) wird in Essigester gelöst und an einer 50 g Kieselgelkolonne chromatographiert. Die Kolonne wird mit einem Gradienten zwischen 4 l Essigester/Hexan (7/3) bis 4 l Essigester und dann 2 l Methylenchlorid/Methylalkohol (8/2) eluiert. Fraktionen von 40 ml werden gesammelt und die Fraktionen Nr. 41-160 werden gepoolt. Das Lösungsmittel wird unter vermindertem Druck entfernt und der erhaltene Rückstand wird in Aethylacetat gelöst und mit einem gleichen Volumen 1N Salzsäure zweimal gewaschen, worauf man zweimal mit einem gleichen Volumen von $Na_2CO_3$ (gesättigt bei Raumtemperatur) wäscht. Die Lösungsmittelphase wird über Natriumsulfat getrocknet. Das Lösungsmittel wird unter vermindertem Druck entfernt. Durch Kristallisation des Rückstandes aus Acetonitril erhält man das Natriumsalz des Antibiotikums X-14873A vom Schmelzpunkt 154°C.

Mikroanalyse: Berechnet für $C_{35}H_{61}O_{11}Na$ (680,87):
Berechnet: %C, 61,73; %H, 9,05; %Na, 3,38
Gefunden: %C, 61,90; %H, 9,01; %Na, 3,11.
$[\alpha]_D^{25}$ -4,5° ($CHCl_3$), 1%), -2,6° ($CH_3OH$, 1%).

## Beispiel 4

Isolierung des Natriumsalzes des Antibotikums X-14873A, sowie der Actinomycine X-14873B, X-14873C und X-14873D as Tankgärungen von Beispiel 2.

Stufe A. Die gesamt Gärlösung von zwei 60 Gallonenfermentern (475 l) wird nach 118 Stunden Wachstum nacheinander zweimal auf ein halbes Volumen Methylenchlorid gegeben. Nach 1-stündigem Rühren wird die Lösungsmittelschicht abgetrennt. Die beiden halben Volumen der Methylenchloridextrakte werden vereinigt und zweimal mit einem halben Volumen 1N Salzsäure, dann mit einem halben Volumen 1N Natronlauge, gefolgt von einem gleichen Volumen von

entionisiertem Wasser gewaschen. Die Lösungsmittelphase wird dann unter vermindertem Druck zu einem Oel konzentriert. Die 336 g Oel werden in n-Hexan gelöst und einmal mit Acetonitril extrahiert. Man erhält einen Niederschlag. Dieser wird in Wasser gelöst und mit einem gleichen Volumen Methylenchlorid extrahiert. Die Methylenchloridphase wird unter vermindertem Druck zur Trockne eingedampft und die erhaltenen 35 g Feststoffe werden in 200 ml Aceton/Acetonitril (60/40) gelöst und in einem Waters Prep LC™/System 500 unter Verwendung von zwei 370 g Prep PAK-500/$C_{18}$ Kolonnen mit Acetonitril/Wasser (80/20) eluiert. Es werden Fraktionen von 200 ml gesammelt und die Fraktionen Nr. 3-5 und 10-14 werden gepoolt.

<u>Stufe B</u>. Der Acetonitrilextrakt von Beispiel 4/Stufe A wird unter vermindertem Druck abgedampft und 18 g des 89 g-schweren Rückstandes werden in 150 ml Acetonitril gelöst und mit einem Waters Prep LC™/System 500 unter Verwendung einer Prep PAK-500/$C_{18}$-Kolonne chromatographiert und mit Acetonitril/Wasser (9:1) eluiert. Es werden 200 ml Fraktionen gesammelt und die Fraktionen Nr. 1-3 und 4-7 werden gepoolt. 71 g des 89 g-schweren Rückstandes des Acetonitrilextrakts werden in Methylalkohol gelöst und an einer Sephadex LH-20-Gelkolonne mit Methylalkohol chromatographiert. Es werden Fraktionen von 40 ml gesammelt und die Fraktionen Nr. 60-200 werden gepoolt. Das Lösungsmittel wird unter vermindertem Druck entfernt und der 45 g-schwere Rückstand wird in Aceton/Acetonitril (60/40) gelöst und dann an einem Waters Prep LC™/System 500 unter Verwendung von zwei 370 g Prep PAK-500/$C_{18}$-Kolonne chromatographiert und mit Acetonitril/Wasser (80/20) eluiert. Es werden Fraktionen von 100 ml gesammelt und die Fraktionen Nrn. 2-4, 5-8, 9-11 und 12-18 werden gepoolt.

<u>Stufe C</u>. Die Fraktionen 10-14 von Beispiel 4/Stufe A, Fraktionen 4-7 und 12-18 von Beispiel 4/Stufe B werden gepoolt. Das Lösungsmittel wird unter vermindertem Druck entfernt und der 19,3 g-schwere Rückstand wird in Methylenchlorid gelöst und an einer 600 g Kieselsäuregelkolonne chromatographiert. Die Kolonne wird mit 3 l Methylenchlorid und dann mit einem Gradienten zwischen 6 l Methylenchlorid bis 6 l Methylenchlorid/Methylalkohol (95/5) eluiert. Es werden Fraktionen von 40 ml gesammelt und die Fraktionen Nrn. 289-360 werden gepoolt. Das Lösungsmittel wird unter vermindertem Druck entfernt, und man erhält nach Kristallisation aus Acetonitril das Natriumsalz des Antibiotikums X-14873A vom Schmelzpunkt 152°C.
Mikroanalyse: Berechnet für $C_{35}H_{61}O_{11}Na$ (680,87):
Berechnet: %C, 61,73; %H, 9,05; %Na, 3,38
Gefunden: %C, 61,08; %H, 9,24; %Na, 3,15.
$[\alpha]_D^{25}$ -5,4° ($CHCl_3$, 1%).

Stufe D. Die Fraktionen 3-5 von Beispiel 4/Stufe A, die Fraktionen 1-3 von Beispiel 4/Stufe B und die Fraktionen 2-4 von Beispiel 4/Stufe B (der zwei HPLC-Läufe) werden gepoolt. Das Lösungsmittel wird unter vermindertem Druck entfernt und die erhaltenen 21 g Rückstand werden mit Diäthyläther verrieben. 14 g Diäthyläther unlösliche Feststoffe werden abfiltriert und in Methylenchlorid gelöst und an einer Kieselgelkolonne chromatographiert. Die Kolonne wird mit einem Gradienten zwischen 6 l Methylenchlorid und 8 l Methylenchlorid/Aethylalkohol/Hexan (95/5/10) und dann mit 2 l Methylenchlorid/Aethylalkohol (95/5) und 2 l Chloroform/Aceton/Methylalkohol (1/1/0,1) eluiert. Es werden Fraktionen von 40 ml gesammelt. Die Fraktionen Nrs. 342-410, 423-466 und 620-646 werden gepoolt. Die vereinigten Fraktionen werden je unter vermindertem Druck konzentriert und liefern nach Zugabe von Diäthyläther orangefarbene Niederschläge von Actinomycin X-14873B (1,4 g), Actinomycin X-14873C (1,2 g) und Actinomycin X-14873D (3,9 g). Actinomycin X-14873B, C und D sind an Silicageldünnschichtplatten unter Verwendung von Methylenchlorid/Aethylalkohol/n-Hexan (9/1/1) unterscheidbar.

Mikroanalyse:

Actinomycin X-14873B; Gefunden %C, 57,42, 57,37; %H, 6,98, 7,00; %N, 12,75, 12,67; $[\alpha]_D^{25}$ -244,5° (MeOH, 1%) -349,6° ($CHCl_3$, 1%).

Actinomycin X-14973C: Gefunden %C, 58,12, 57,97; %H, 7,06, 7,01; %N, 13,00, 12,95; $[\alpha]_D^{25}$ -299,1° (MeOH, 1%) -395,2° ($CHCl_3$, 1%).

Actinomycin X-14873D: Gefunden %C, 56,50, 56,49; %H, 6,80, 7,00; %N, 12,06, 12,16; $[\alpha]_D^{25}$ +11° (MeOH, 0,1%), -145,5° ($CHCl_3$, 1%).

Massenspektralanalyse durch Bombardement mit schnellen Atomen der Actinomycine X-14873C und X-14873D bestätigte, dass die zwei Antibiotika isomer sind und eine Summenformel von $C_{61}H_{86}N_{12}O_{18}$ aufweisen mit einem Molekulgewicht von 1275,44 (berechnet %C, 57,44; %H, 6,80; %N, 13,18). Diese Ergebnisse bestätigen die Neuheit von X-14873B und D, welche - ähnlich wie X-14873B - bei der Hydrolyse unter anderm beide Prolin und 3-Hydroxy-5-methylprolin ergeben, wodurch all diese drei Formen klar von bekannten Actinomyces unterscheidbar sind.

Die Aktivitäten der drei Actinomyces sind:

| Antibiotikum | Akute Toxizität an der Maus $LD_{50}$ (mg(kg) | | Antitumor gegen S-180 Tumorimplantat an Mäusen (mg/kg) | |
|---|---|---|---|---|
| | i.p. | p.o. | Aktive Spiegel | Toxische Spiegel |
| Actinomycin | | | | |
| X-14873B | 0,16 | 2,9 | 0,048 | 0,096 |
| X-14873C | | >1000 | | 6,25 |
| X-14873D | 71 | >1000 | 6-12 | 25 |

Zusätzlich war Actinomycin X-14873B in vivo aktiv als Coccidiostatikum in Geflügel. Dies ist das erste Mal, dass eine solche Wirksamkeit für ein Actinomycin beobachtet wurde.

## Beispiel 5

Die das Streptomyces X-14873 erzeugende Kultur wird auf einer Stärke-Caseinagarschrägkultur folgender Zusammensetzung (g/l in destilliertem Wasser) gezüchtet und aufbewahrt:

| | |
|---|---|
| Lösliche Stärke | 10,0 |
| Casein | 1,0 |
| $K_2HPO_4$ | 0,5 |
| $MgSO_4$ (wasserfrei) | 0,5 |
| Agar | 20,0 |

Der pH des Mediums wird vor der Autoklavierung mit NaOH auf 7,4 eingestellt.

Die Schrägkultur wird mit X-14873 beimpft und bei 28°C während 7-14 Tagen inkubiert. Ein Teil des Agars enthaltend Sporen und Mycel aus der gut gewachsenen Agarkultur wird zur Beimpfung eines 500 ml-Erlenmeyerkolbens mit 100 ml sterilisiertem Impfsubstrat folgender Zusammensetzung (g/l in destilliertem Wasser) verwendet:

| | |
|---|---|
| Tomatenmark | 5,0 |
| getrocknete Rückstände aus der Brennerei | 5,0 |
| OM Pepton | 5,0 |
| entbitterte Hefe | 5,0 |
| Eclipse N Stärke | 20,0 |
| $CaCO_3$ | 1,0 |
| $K_2HPO_4$ | 1,0 |

Der pH-Wert wird vor der Sterilisierung auf 7 eingestellt.

Die beimpfte Nährlösung wid bei 28°C während 4 Tagen auf einer Schüttelmaschine (250 Upm) inkubiert.

Eine 10 ml-Portion dieser Kultur wird zur Beimpfung eines 6 l-Erlenmeyerkolbens enthaltend 2 l sterilisiertes Impfsubstrat der oben erwähnten Zusammensetzung verwendet.

Die beimpfte Nährlösung wird bei 28°C während 3 Tagen auf einer Schüttelmaschine (250 Upm) inkubiert.

6 l der erhaltenen Kulturbrühe werden dann zur Beimpfung von 230 l Nährmedium in einem 380 l Fermenter verwendet, wobei das Impfmedium die oben beschriebene Zusammensetzung aufweist. Das Impfmedium im Fermentor wird mit 0,1 g/l Antischaummittel SAG 4130 (Union Carbide) ergänzt und während 1 1/4 Stunden mit Dampf von 4,2 $kg/cm^2$ sterilisiert.

Das beimpfte Medium im Fermentor wird mit Druckluft von einer Geschwindigkeit von 90 l/Minute belüftet und mit einem Rührwerk (220 Upm) gerührt. Nach 3 Tagen Inkubierung werden 11 Gallonen der resultierenden Fermentationsbrühe zur Beimpfung von 360 Gallonen des folgenden Mediums (in g/l Leitungswasser) in einem 500 Gallonen Fermentor verwendet:

| | |
|---|---|
| Cerelose | 20,0 |
| Pepton | 5,0 |
| NaCl | 5,0 |
| $CaCO_3$ | 2,0 |
| Natriumpropionat | 1,0 |
| SAG 4130 Antischaummittel (Union Carbide) | 0,1 |

Das beimpfte Medium im Fermentor wird pro Minute mit Druckluft von 1,4 $kg/cm^2$ belüftet und mit einem Rührwerk (280 Upm) gerührt. Die Fermentation wird während 139 Stunden bei 28°C durchgeführt.

## Beispiel 6

Isolierung des Antibiotikums X-14873G aus Tankgärungen von Streptomyces Kultur X-14873.

Stufe A. Die ganze Gärlösung von 1330 l nach 139 Stunden Gärzeit wird mit der gleichen Menge Essigester versetzt. Nach 1-stündigem Rühren wird die Lösungsmittelschicht abgetrennt und unter vermindertem Druck auf 5,75 l eingedampft. Der konzentrierte Extrakt wird mit 1N Salzsäure, gesättigter Natriumcarbonatlösung und entionisiertem Wasser gewaschen.

Die Lösungsmittelphase wird konzentriert bis das rohe Natriumsalz des Antibiotikums X-14873 auskristallisiert. Die Mutterlauge wird unter vermindertem Druck zu einem Oel konzentriert. Das Oel wird in Methylenchlorid gelöst und mit Methylenchlorid an einer 4 kg-Kieselgelkolonne chromatographiert. Die Kolonne wird mit einem Gradienten zwischen 2 l Methylenchlorid und 6 l Hexan/Methylenchlorid (1/1) gefolgt von 8 l Hexan-Aceton (9/1) eluiert. Es werden Fraktionen von 500 ml gesammelt, und die Fraktionen Nrn. 1-4 und 5-8 werden gepoolt.

Stufe B. Die gepoolten Fraktionen 1-4 von Stufe A werden unter vermindertem Druck zur Trockne gedampft und der Rückstand von 81 g wird in Methylenchlorid gelöst und an einer 700 g Kieselgelkolonne chromatographiert. Die Kolonne wird mit einem Gradienten zwischen 3 l Methylenchlorid/Hexan (1/1) bis 4 l Hexan/Aceton (95/5) gefolgt von 4 l Hexan/Aceton (9/1) eluiert. Es werden Fraktionen von 40 ml gesammelt und die Fraktionen Nrn. 52-310 und 320-390 werden gepoolt. Das Lösungsmittel wird unter vermindertem Druck abgedampft und aus den gepoolten Fraktionen 320-390 kristallisiert aus Diäthyläther/Hexan das Antibiotikum X-14873G vom Schmelzpunkt 152-173°C.
Mikroanalyse: Berechnet für $C_{34}H_{60}O_8$ (596,82):
Berechnet: %C, 68,42, %H, 10,13
Gefunden: %C, 68,28; %H, 10,10; $[\alpha]_D^{25}$ +5,5° ($CHCl_3$, 1%).

Stufe C. Die gepoolten Fraktionen 5-8 von Stufe A werden unter vermindertem Druck zur Trockene eingedampft, und der Rückstand (80 g) wird in Methylenchlorid gelöst und an einer 1 kg Kieselgelkolonne chromatographiert. Die Kolonne wird mit einem Gradienten zwischen 4 l Methylenchlorid und 8 l Hexan/Aceton (9/1) gefolgt von 6 l Hexan/Aceton (8/2), 3 l Hexan/Aceton (6/4) und 3 l Methylenchlorid/Aceton (8/2) eluiert. Es werden Fraktionen von 40 ml gesammelt. Die Fraktionen Nrn. 116-160, 161-182 und 183-270 werden gepoolt, und das Lösungsmittel wird unter vermindertem Druck abgedampft. Durch Kristallisation aus Methylenchlorid/Hexan des Rückstandes der Fraktionen 115-160 erhält man zusätzliches Antibiotikum X-14873G. Durch Kristallisation von Methylenchlorid/Hexan des Rückstandes der Fraktionen 183-270 erhält man Antibiotikum X-14873H vom Schmelzpunkt 145-146°C.
Mikroanalyse berechnet für $C_{34}H_{62}O_9$ (614,84):
Berechnet: %C, 66,41; %H, 10,16
Gefunden: %C, 66,43; %H, 9,92
66,55 9,95;
$[\alpha]_D^{25}$ -5,3° ($CHCl_3$, 1%) +3,6° ($CH_3OH$, 1%).

Die gepoolten Fraktionen 161-182 enthalten ein Gemisch der Antibiotika X-14873G und -H.

## Beispiel 7

Herstellung des Thalliumsalzes des Antibiotikums X-14873A.

Eine Lösung des Natriumsalzes des Antibiotikums

X-14873A in Methylenchlorid wird mit 1N Salzsäure, gefolgt von Wasser und dann viermal mit einer wässrigen Lösung von Thalliumhydroxid gewaschen. Die Lösungsmittelphase wird konzentriert, und durch Zugabe von n-Hexan wird das kristalline Thalliumsalz vom Antibiotikum X-14873A gewonnen.
Schmelzpunkt 154°C.
Mikroanylse: Berechnet für $C_{35}H_{61}O_{11}Tl$ (862,25):
Berechnet: %C, 48,76; %H, 7,13; %Tl, 23,7
Gefunden: %C, 49,01; %H, 7,07; %Tl, 21,91.

Die Struktur des Antibiotikums X-14873A wird durch Röntgenstrahlanalyse des Thalliumsalzes bestimmt.

## Beispiel 8

Herstellung des Calciumsalzes des Antibiotikums X-14873A.

Eine Lösung des Natriumsalzes des Antibiotikums X-14873A in Methylenchlorid wird mit 1N Salzsäure, gefolgt von wässriger Calciumhydroxidlösung und Wasser gewaschen. Die Lösungsmittelphase wird unter vermindertem Druck konzentriert und kristallisiert durch die Zugabe von n-Hexan.
Schmelzpunkt 133°C.
Mikroanalyse: Berechnet für $(C_{35}H_{61}O_{11})_2Ca$ (1355,84):
%C, 62,01; %H, 9,07; %Ca, 2,96
Gefunden: %C, 62,17; %H, 9,28; %Ca, 2,96;
$[\alpha]_D^{25}$ 3,5° (MeOH, 1%), 9,99° ($CHCl_3$, 1%).

Antibiotikum X-14973A zeigte eine orale Toxizität ($LD_{50}$) in Mäusen von 120 mg/kg (24 Stunden); X-14873G zeigte eine orale Toxizität ($LD_{50}$) in Mäusen von 4000 mg/kg (24 Stunden) und X-14873H zeigte eine orale Toxizität ($LD_{50}$) in Mäusen von 4000 mg/kg (24 Stunden).

Diese Antibiotika zeigen - obwohl begrenzt - eine Aktivität gegen eine Vielzahl von Bakterien und Dermatophyten wie dies in den nachfolgenden Tabellen zum Aus-

druck kommt.

| Mikroorganismus | Mindesthemmkonzentration (mcg/ml) - X-14873A |
|---|---|
| Staphylococcus aureus Smith | 64 |
| Escherichia coli 257 | 128 |
| Salmonella typhi P58A | 128 |
| Klebsiella pneumoniae A | 128 |
| Proteus mirabilis 190 | 128 |
| Pseudomonas aeruginosa B | 128 |
| Serratia marcescens SM | 128 |
| Enterobacter cloacae 5699 | 128 |
| Trichophyton mentagrophytes | 100 |
| Microsporum audouini | 100 |

| Name des Mikroorganismus | ATCC Nummer | Mindesthemmkonzentration (mcg/ml) | | |
|---|---|---|---|---|
| | | X-14873A | X-14873H | X-14873G |
| Streptococcus faecium | 8043 | 0.1 | 2 | 63 |
| Staphylococcus aureus | 6538P | 6.3 | 8 | > 1000 |
| Sarcina lutea | 9341 | 3.0 | 16 | > 1000 |
| Bacillus megaterium | 8011 | 3.0 | 4 | > 1000 |
| Bacillus sp. E | 27359 | 0.8 | 2 | 8 |
| Bacillus subtilis | 558* | 3.0 | 16 | > 1000 |
| Bacillus sp. TA | 27860 | 3.0 | 8 | > 1000 |
| Mycobacterium phlei | 355 | 3.0 | > 1000 | > 1000 |
| Streptomyces cellulosae | 3313 | 12.5 | > 1000 | > 1000 |
| Paecilomyces varioti | 25820 | 125 | > 1000 | > 1000 |
| Penicillum digitatum | 26821 | 500 | > 1000 | > 1000 |
| Candida albicans | 477* | 62.5 | > 1000 | > 1000 |
| Saccharomyces cerevisiae | 4226 | 500 | > 1000 | > 1000 |

* NRRL Nummer

Wie oben erwähnt besitzen die Antibiotika A, G und H die Eigenschaft, das Wachstum von gewissen gram-positiven Bakterien negativ zu beeinflussen. Sie sind deshalb verwendbar als Waschlösungen für sanitäre Zwecke, zum Händewaschen und zur Reinigung von Geräten, Böden und Einrichtungen von kontaminierten Räumen und Laboratorien.

Das Antibiotikum X-14873A ist wirksam als wachstumförderndes Mittel bei Widerkäuern, d.h. Tieren mit einer Pansenfunktion, z.B. Rindvieh. Eine Erläuterung des Mechanismus, wie das Futter in widerkäuenden Tieren verdaut abgebaut und metabolisiert wird, wird in der U.S. Patentschrift Nr. 3,839,557 gegeben; es wird dort die Verwendung gewisser Antibiotika zur Verbesserung des Futternutzeffektes bei Widerkäuern beschrieben. Wirtschaftlich wichtige Widerkäuer sind Rind, Schaf und Ziege.

Es wird Pansenflüssigkeit eines Stieres mit einer Pansenfistel gewonnen. Der Stier erhält folgendes Futter:

Mais: 89,93%
Alfalfamehl: 5,000%
Soyabohnenöl: 3,00%
Kalk: 0,80%
NaCl: 0,60%
Dicalciumphosphat: 0,50%
Spurenmineralien: 0,025%
Vitamin-Prämix-Zusätze: 0,1%
  Vitamin A, TIU: 4,0003
  Vitamin $D_3$, IU: 0,801
  Vitamin E, TIU: 3,002.

Die Pansenflüssigkeit wird unmittelbar durch ein Sieb gesiebt. Die Flüssigkeit wird wie folgt einer Fermentation unterworfen:

Für jede Fermentation werden 75 ml der erhaltenen Flüssigkeit in einen 250 ml-Kolben gegeben, der ausserdem

folgende Zusätze enthält:

1 g 80%:20% fein gemahlenes Korn/Heu;

1 ml einer 18%igen wässrigen Glucoselösung (1 mMol) pro Kolben);

1,5 ml einer 3,1%igen wässrigen Harnstofflösung (0,76 Mol pro Kolben);

Je 60 mMol der 10 essentiellen Aminosäuren (Arginin, Histidin, Leucin, Methionin, Threonin, Valin, Lysin, Isoleucin, Phenylalanin, Tryptophan);

1 ml einer wässrigen Lösung der Testsubstanz, um entweder 10 oder 25 µg/ml zu erhalten (berechnetes totales Volumen der Fermentationsmischung: 80 ml).

Jeder Kolben wird bei 38°C in einem mit Abzug versehenen, geschüttelten Wasserbad bebrütet. Kohlendioxid wird kontinuierlich durch den Abzug geleitet. Nach 4-stündiger Inkubation werden 10 ml der Fermentationsflüssigkeit 20 Minuten bei 14000 Upm (etwa 30000 g) zentrifugiert. 1 ml einer 25%igen Methaphosphorsäurelösung, enthaltend 23 mMol 2-Methylvaleriansäure wird mit 3 ml der aus der Zentrifugierung erhaltenen überstehenden Phase versetzt. Die erhaltene Flüssigkeit wird bei Zimmertemperatur 30 Minuten stehengelassen. Die Flüssigkeit wird durch ein 0,22 Millimicron-Filter filtriert und bis zur gaschromatographischen Analyse der flüchtigen Fettsäuren in der Kälte aufbewahrt.

Gas-Flüssigchromatographische Analysen von einer nichtbehandelten Kontrollfermentation und zwei Fermentationen eine mit Antibiotikum X-14873A und die andere mit Lasalocid werden in der folgenden Tabelle angegeben. Die Antibiotika sind in den Rationen in einer Konzentration von 30 g/Tonne eingeschlossen.

| Experiment Tag | Verhältnis der flüchtigen Fettsäuren:[a] $C_3/(C_2 + nC_4)$ Experimentelle Gruppen nicht behadelt[b] | Lasalocid[c] 30 g/Tonne | X-14873A[c] 30 g/Tonne |
|---|---|---|---|
| Vormedikation | | | |
| 1 | 0,262±0,065 | 0,229±0,013 | 0,213±0,016 |
| 3 | 0,260±0,050 | 0,255±0,051 | 0,235±0,036 |
| 5 | 0,240±0,024 | 0,244±0,060 | 0,234±0,042 |
| 8 | 0,240±0,042 | 0,236±0,041 | 0,245±0,046 |
| 10 | 0,206±0,037 | 0,224±0,011 | 0,216±0,038 |
| Medikation | | | |
| 2 | 0,211±0,019 | 0,376±0,109 | 0,445±0,075 |
| 5 | 0,248±0,058 | 0,390±0,073 | 0,584±0,328 |
| 7 | 0,325±0,166 | 0,373±0,015 | 0,679±0,448 |
| 9 | 0,338±0,164 | 0,381±0,064 | 0,642±0,387 |
| 12 | 0,293±0,148 | 0,372±0,056 | 0,495±0,242 |

(a) ± eine Standardabweichung
(b) 4 Tiere
(c) 3 Tiere

Die obige Tabelle zeigt, dass das Verhältnis von Propionat ($C_3$) zu Acetat ($C_2$) und n-Butyrat ($nC_4$) wesentlich verbessert wird. Bei vermehrter Bildung von Propionat im Gegensatz von Acetat aus den Kohlenhydraten wird die Wirkung der Kohlenhydrate und daher die Futterverwertung erhöht. Aus der Tabelle geht weiter hervor, dass das Antibiotikum X-14873A eine grössere Zunahme des Verhältnisses der flüchtigen Fettsäuren gegenüber Kontrollen und gegenüber Lasalocid, einer aktiven Verbindung, bewirkt.

Durch Verabreichung des Antibiotikums X-14873A, (nachstehend "das Antibiotikum" genannt) wird Ketose verhindert bzw. geheilt; gleichzeitig wird die Futterverwertung verbessert. Die Ursache der Ketose liegt in einer ungenügenden Bildung von Propionat. Eine gegenwärtige

empfohlene Behandlungsmethode besteht in der Verabreichung von Propionsäure bzw. von Futtern, welche bevorzugt Propionat erzeugen. Es ist offensichtlich, dass die vermehrte Bildung von Propionat bei der Einnahme von üblichen Futtermitteln die Ketosefälle vermindern wird.

Es wurde gefunden, dass das Antibiotikum X-14873A die Wirksamkeit der Futterverwertung erhöht, wenn es den Tieren oral verabreicht wird. Die einfachste Methode zur Verabreichung des Antibiotikums besteht darin, dass man es mit dem Tierfutter vermischt.

Das Antibiotikum kann jedoch auch in anderer Weise verabreicht werden. Es kann beispielsweise zu Tabletten, Tränken, Boli oder Kapseln verarbeitet werden, welche dem Tier in dosierter Form verabreicht werden können. Formulierungen des Antibiotikums in derartigen Dosierungsformen werden nach an sich in der Veterinärpharmazie bekannten Methoden hergestellt.

Kapseln werden in üblicher Weise hergestellt durch Auffüllen von Gelatinekapseln beliebiger Form mit dem Antibiotikum. Erwünschtenfalls kann das Antibiotikum mit einem inerten, festen Verdünnungsmittel verdünnt werden, wie beispielsweise mit einem Zucker, Stärke oder gereinigter kristalliner Cellulose, um das Volumen aus Zweckmässigkeitsgründen zu erhöhen.

Tabletten werden durch herkömmliche pharmazeutische Methoden hergestellt. Eine Tablette enthält im allgemeinen ausser dem Wirkstoff einen Grundstoff, einen Desintegrator, ein Absorbens, ein Bindemittel und ein Gleitmittel. Typische Grundstoffe sind z.B. Milchzucker, feiner Puderzucker, Natriumchlorid, Stärke und Mannit. Stärke ist ebenfalls ein guter Desintegrator, wie ebenso Alginsäure. Oberflächenaktive Mittel, wie Natriumlaurylsulfat und Dioctylnatriumsulfosuccinat werden gelegentlich ebenfalls verwendet. Ueblicher Weise verwendete Absorbentien sind z.B. Stärke

und Milchzucker, während Magnesiumcarbonat bei öligen Substanzen Verwendung findet. Oft verwendete Bindemittel sind Gelatine, Gummi arabicum, Stärke, Dextrin und verschiedene Cellulosederivate. Häufig verwendete Gleitmittel sind z.B. Magnesiumstearat, Talk, Paraffinwachs, verschiedene Metallseifen und Polyäthylenglykol.

Das Antibiotikum kann ebenfalls in Form eines Bolus mit verzögerter Wirkung verabreicht werden. Derartige Boli werden als Tabletten hergestellt, wobei jedoch ein Mittel zur Verzögerung der Auflösung des Antibiotikums zugefügt wird. Boli sind für Freisetzung während längerer Perioden vorgesehen. Die langsame Auflösung wird durch Wahl einer stark wasserunlöslichen Form des Antibiotikums gewährleistet. Beispielsweise können Eisenfeilenspäne zugegeben werden, um die Dichte des Bolus zu erhöhen, damit dieser am Boden des Pansens bleibt.

Die Auflösung des Antibiotikums wird durch Verwendung einer Matrize unlöslichen Materials, in welcher der Wirkstoff eingebettet wird, verzögert. Beispielsweise können pflanzliche Wachse, gereinigte Mineralwachse und wasserunlösliche, polymere Materialien verwendet werden.

Tränke des Antibiotikums werden zweckmässig durch Wahl einer wasserlöslichen Form des Antibiotikums hergestellt. Wird eine unlösliche Form gewünscht, kann eine Suspension hergestellt werden. Wahlweise kann ein Trank aus einer Lösung in einem physiologisch annehmbaren Lösungsmittel, wie beispielsweise Polyäthylenglykol, zubereitet werden.

Suspensionen von unlöslichen Formen des Antibiotikums können in nicht-Lösungsmitteln hergestellt werden, wie beispielsweise in pflanzlichen Oelen, z.B. Erdnuss-, Mais- oder Sesamöl, in einem Glykol, wie z.B. Propylenglykol oder Polyäthylenglykol, oder in Wasser, je nach der gewählten Form des Antibiotikums.

Geeignete physiologisch annehmbare Zusätze sind notwendig, um das Antibiotikum in Suspension zu halten. Die Zusätze können Verdickungsmittel sein, wie z.B. Carboxymethylcellulose, Polyvinylpyrrolidon, Gelatine oder Alginate. Verschiedene Klassen von oberflächenaktiven Stoffen dienen dazu, dass Antibiotikum in Suspension zu erhalten. Beispielsweise sind Lecithin, Alkylphenol/Polyäthylenoxidaddukte, Naphthalinsulfonate, Alkylbenzolsulfonate und Polyoxyäthylensorbitanester für die Herstellung von Suspensionen in flüssigen Nichtlösungsmitteln verwendbar.

Für die Herstellung von Suspensionen können zusätzlich verschiedene Substanzen, welche die Hydrophilität, Dichte und Oberflächenspannung beeinflussen, verwendet werden, Beispiele sind Antischaumsilikole, Glykole, Sorbit und Zucker, die als Suspendierungsmittel Verwendung finden.

Das Antibiotikum kann dem Züchter als eine Suspension oder als trockenes Gemisch mit entsprechenden Hilfsmitteln zur Verdünnung vor der Verwendung angeboten werden.

Das Antibiotikum kann auch dem Trinkwasser von Wiederkäuern zugeführt werden. Dabei wird eine wasserlösliche oder wassersuspendiertbare Form des Antibiotikums dem Trinkwasser in einer entsprechenden Menge zugegeben. Die Zubereitung des Antibiotikums für das Vermischen mit dem Trinkwasser folgt den gleichen Prinzipien wie die Formulierung von Tränken.

Die zweckmässigste Art, das Tier mit dem Antibiotikum zu behandeln, basiert auf der Verabreichung mit dem Tierfutter. Beliebige Tierfutter können verwendet werden, z.B. übliche Trockenfutter, flüssige Futter und tablettierte Futter.

Die Methoden und Arzneimittel in Tierfutter einzubringen sind bestens bekannt. Zur Herstellung eines solchen Tierfutters wird üblicher Weise zuvor eine konzentrierte

Vormischung mit dem Antibiotikum hergestellt. Beispielsweise kann eine Vormischung von etwa 2 bis etwa 900 g Antibiotikum pro Kilo Vormischung enthalten. Dieser weite Bereich ist durch den benötigten, weiten Konzentrationsbereich für das fertige Futter bedingt. Vormischung können sowohl in flüssiger als auch in fester Form vorliegen.

Die Formulierung von Widerkäuerfuttern enthaltend die genaue Menge von Antibiotikum für eine übliche Behandlung ist bestens bekannt. Es ist nur notwendig, den Anteil einer Verbindung, welche für die Verabreichung jedes einzelnen Tieres erwünscht ist, zu berechnen und den Anteil an Futter pro Tag, welches das Tier frisst und die Konzentration des Antibiotikums in der Vormischung zu berücksichtigen und dann die genaue Konzentration des Antibiotikums oder der Vormischung im Futter zu berechnen.

Alle Methoden zur Formulierung, Mischung und Tablettierung von Futter, welche üblicher Weise bei Widerkäuernfutter verwendet werden, sind zur Herstellung von Futtern enthaltend das Antibiotikum geeignet.

Wie oben gezeigt, bewirkt die orale Verabreichung des Antibiotikums eine vorteilhafte Aenderung der Erzeugung von Propionat im Verhältnis zu Acetat in Pansen. Es kann deshalb postuliert werden, dass die gleiche Behandlung ebenfalls monogastrische Tiere, die faseriges Pflanzenmaterial im Blinddarm vergären, günstig beeinflusst, denn es kann erwartet werden, dass eine vorteilhafte Veränderung in Propionat-Acetat-Verhältnis nach oraler Gabe des Antibiotikums auftreten wird. Pferde, Schweine und Kaninchen sind Beispiele von Tieren, welche einen Teil ihres Futters durch Fermentation im Blinddarm verdauen.

0088446

Das Antibiotikum X-14873A ist ebenfalls wirksam als Mittel bei der Behandlung bzw. Prophylaxe bei Schweinedysenterie. Die Wirkung der Verbindung gegen Treponema hyodysenteriae, den Erreger von Schweinedysenterie, wurde untersucht. Die Verbindung zeigt eine minimale Hemmkonzentration von 5 mcg/ml.

Das Antibiotikum X-14873 zeigt ebenfalls eine Aktivität als Coccidiostatikum und zwar zeigt es in vitro eine Aktivität gegen E. tenella bei 200 ppm.

## Patentansprüche

1. Verbindung X-14873A, G und H der Formel

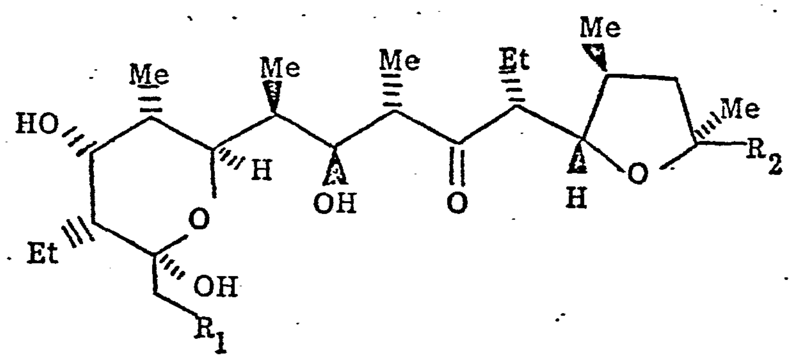

I

worin für X-14873A, $R_1$ $CO_2H$ und $R_2$

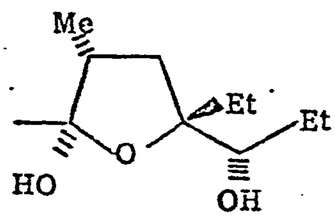

;

ist,

für X-14873G $R_1$ Wasserstoff und $R_2$

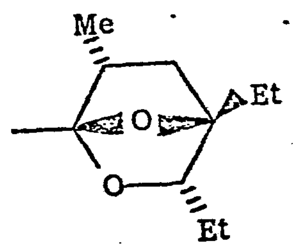

;

ist,

und für X-14873H, $R_1$ Wasserstoff und $R_2$

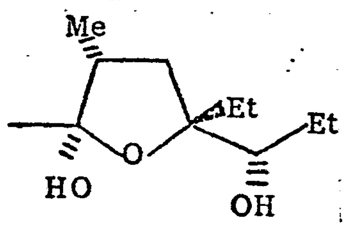

ist,

und worin Me für Methyl und Et für Aethyl steht, und pharmazeutisch annehmbare Salze davon.

2. Biologisch reine Kultur von Streptomyces sp. X-14873.

3. Verbindungen gemäss Anspruch 1 als Antibiotika.

4. Verbindungen gemäss Anspruch 1 als pharmazeutisch aktive Substanzen gegen eine Vielzahl von Bakterien und Dermatophyten.

5. Verbindungen gemäss Anspruch 1 als Coccidiostatika.

6. Verbindung X-14873A gemäss Anspruch 1 als pharmazeutisch aktiver Wirkstoff für die Behandlung und Prophylaxe der Schweinedysenterie.

7. Verbindung X-14873A gemäss Anspruch 1 als wachstumförderndes Mittel bei Widerkäuern.

8. Verfahren zur Herstellung der Verbindungen X-14873A, G und H gemäss Anspruch 1, dadurch gekennzeichnet, dass man einen Stamm von Streptomyces sp. X-14873 in einer kohlehydrat-und stickstoffhaltigen Nährstoffe enthaltenden wässrigen Lösung unter submersen aeroben Bedingungen kultiviert und die Produkte aus der Lösung isoliert.

9. Antibiotische Zubereitung enthaltend eine Verbindung gemäss Anspruch 1.

10. Pharmazeutische Zubereitung gegen eine Vielzahl von Bakterien und Dermatophyten enthaltend eine Verbindung gemäss Anspruch 1.

11. Pharmazeutische Zubereitung für die Behandlung von Prophaxlase der Coccidiose enthaltend eine Verbindung gemäss Anspruch 1.

12. Pharmazeutische Zubereitung für die Behandlung und Prophylase der Schweinedysenterie enthaltend Verbindung X-14873A gemäss Anspruch 1.

0088446

13. Vormischung oder Komposition zum unmittelbaren Verbrauch als wachstumförderndes Mittel bei Widerkäuern, enthaltend die Verbindung X-14873A gemäss Anspruch 1.

## Patentanspruch für Oesterreich

Verfahren zur Herstellung der Verbindungen X-14873A, G und H der Formel

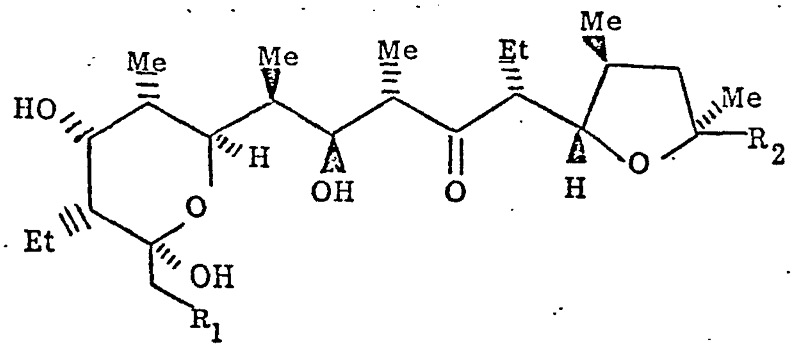 I

worin für X-14873A $R_1$ $CO_2H$ und $R_2$

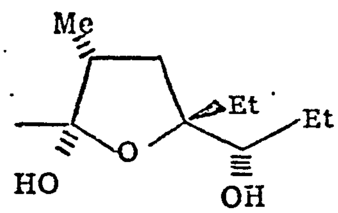 ;

ist,
für X-14873G $R_1$ Wasserstoff und $R_2$

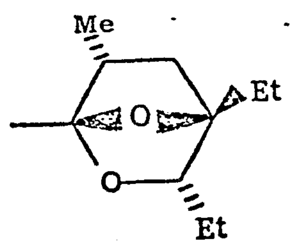 ;

ist,
und für X-14873H $R_1$ Wasserstoff und $R_2$

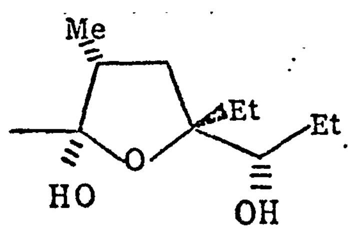

ist,
und worin Me für Methyl und Et für Aethyl steht, sowie von pharmazeutisch annehmbaren Salzen davon, dadurch

gekennzeichnet, dass man einen Stamm von Streptomyces sp. X-14873 in einer kohlehydrat- und stickstoffhaltigen Nährstoffe enthaltenden wässrigen Lösung unter submersen aeroben Bedingungen kultiviert und anschliessend die Verbindungen von dieser Lösung isoliert und falls erwünscht diese Verbindungen in pharmazeutisch annehmbare Säureadditionssalze davon überführt.